# EUROPEAN PATENT APPLICATION

(11) **EP 4 846 931 A1**
(43) Date of publication of application: **02.12.2026**
(21) Application number: 26179254.3
(22) Date of filing: 18.05.2026
(51) Int. Cl.: A61B 5/021, A61B 5/022, A61B 5/0225, A61B 5/0245, A61B 5/352, A61B 5/00

(54) **GATING BLOOD PRESSURE MEASUREMENTS BY A SECONDARY SIGNAL**

(30) Priority: 29.05.2025 US 202563814222 P; 11.05.2026 US 202619673412
(71) Applicant: Stryker Corporation, Portage, MI 49002 (US)
(72) Inventor: SIEDENBURG, Clinton T., Portage, 49002-9711 (US); LEE, Frank Jian, Portage, 49002-9711 (US); BAYDOUN, Habib Imad, Portage, 49002-9711 (US)
(74) Representative: Kilburn & Strode LLP

(57) **Abstract**

An example method includes identifying data indicating a pressure in a cuff configured to be disposed around a limb of a subject; identifying data indicating a secondary parameter indicative of pulses in the limb of the subject; determining, by analyzing the data indicating the secondary parameter, one or more pulse intervals of the pulses in the limb of the subject; and identifying a portion of the data indicating the pressure in the cuff corresponding to the one or more pulse intervals. The example method further includes determining a blood pressure of the subject by analyzing the portion of the data indicating the pressure in the cuff corresponding to the one or more pulse intervals. In addition, the example method includes causing an output device to output an indication of the blood pressure.

## Description

### BACKGROUND

Blood pressure is an important vital sign that is indicative of a patient's health. Clinically, blood pressure often refers to both a systolic pressure (SBP), which refers to the maximum pressure measured during the cardiac cycle (e.g., one heartbeat), as well as a diastolic pressure (DBP), which refers to a minimum pressure between multiple cardiac cycles. Other clinically relevant blood pressure metrics include mean arterial pressure (MAP) and pulse pressure. Both MAP and pulse pressure (PP) are related to systolic and diastolic blood pressure.

Blood pressure can be invasively measured using a catheter inserted into the patient's circulatory system. It can also be measured noninvasively by inflating a blood pressure cuff. The blood pressure cuff may be inflated in order to squeeze an extremity of the patient. Typically, during deflation of the blood pressure cuff the pressures are observed at which systolic and/or diastolic pressures are reached.

### BRIEF DESCRIPTION OF THE DRAWINGS

FIG. 1 illustrates an example environment for determining blood pressure of a subject using the blood pressure measurement gating techniques disclosed herein.
FIG. 2 illustrates example pulse time delays at different sensors deployed on a subject.
FIG. 3 illustrates example pulse data, adjusted pulse data which accounts for a delay, and pressure data which is gated using the adjusted pulse data.
FIG. 4A illustrates an example pressure waveform that can be generated using various techniques described herein.
FIG. 4B illustrates an example cuff pressure curve, a section of the cuff pressure curve bounded by Korotkoff sounds, an oscillometric waveform that can be extracted from the cuff pressure curve and optionally gated according to the techniques described herein, and an oscillometric waveform envelope that can be constructed from the oscillometric waveform.
FIG. 5 illustrates example secondary data including features corresponding to likely artifacts, and example pressure data which can be adjusted by removing portions identified using the secondary data.
FIG. 6 illustrates an example process for gating blood pressure measurements by a secondary signal to noninvasively determine the blood pressure of a subject.
FIG. 7 illustrates another example process for gating blood pressure measurements by a secondary signal to noninvasively determine the blood pressure of a subject.
FIG. 8 illustrates an example of an external defibrillator configured to perform various functions described herein.

### DETAILED DESCRIPTION

Existing techniques for blood pressure detection have a number of drawbacks. For example, invasive blood pressure measurements are less practical and more dangerous than other blood pressure measurement techniques in out-of-hospital environments. Invasive blood pressure measurements also carry inherent risks for patients, even in controlled clinical environments, such as hospitals.

Further, noninvasive cuff-based blood pressure measurements are prone to error, particularly when the patient or the cuff is in motion. Error can lead to repeated measurement attempts, which are uncomfortable for patients. In some cases, a successful and accurate blood pressure measurement using existing noninvasive blood pressure techniques may be unachievable in out-of-hospital environments.

Various implementations described herein relate to gating blood pressure measurements by a secondary signal in order to remove potential artifacts from the blood pressure measurements. By removing potential artifacts, the accuracy and reliability of the blood pressure measurements can be improved. In an example, blood pressure measurements can be noninvasively collected using a blood pressure cuff. The secondary signal can include, e.g., an electrocardiogram (ECG) signal from which pulse timing information can be derived and used to determine pulse timing at the blood pressure cuff. The pulse timing at the blood pressure cuff can in turn be used to gate the blood pressure measurements. Various other secondary signals can also be used to gate blood pressure measurements, instead of or in addition to the ECG signal. Furthermore, the secondary signal(s) can also be used for various other purposes described herein.

Automated non-invasive blood pressure (NIBP) measurements conducted without the benefit of the techniques described herein can be unreliable and/or inaccurate, especially in uncontrolled conditions such as experienced by first responders administering emergency care. One driver for poor quality blood pressure measurements in emergency care environments is the susceptibility of NIBP modules to noise and vibration due to motion.

An issue that can cause both inaccurate readings and/or no reading at all from an automated NIBP monitor is the corruption of the blood pressure signal from external noise. In the emergency care environment, the sources of noise that could corrupt/disturb a NIBP measurement are numerous, a few notable sources being vibration while in an emergency transport vehicle (e.g., an ambulance and/or cot) and body movements of unstable patients.

NIBP measurements, in some cases, include detecting the pressure within a cuff that is inflated around a limb of a subject over multiple cardiac cycles. The cardiac cycles, for instance, create oscillations in the blood pressure within the limb that result in corresponding oscillations in the waveform representing the detected pressure over time. Accordingly, the pressure over time within the cuff can be referred to as an oscillometric measurement. One reason motion artifacts can negatively impact the accuracy of oscillometric NIBP measurements is because the accuracy of oscillometric NIBP processing often relies on the quality of the patient pulse data or oscillation (cuff volumetric and pressure change). Motion artifacts can distort the patient pulse data that could render oscillometric measurement inaccurate or completely invalid, depending on how excessive the motion is. It is challenging to filter out motion artifacts in part because the frequency bandwidth of motion artifacts may overlap with that of the oscillometric signal (e.g., both may have frequencies in the 1 - 10 Hertz (Hz) range).

Blood pressure measurements made via a pressure sensor in a blood pressure cuff are therefore susceptible to corruption by spurious artifacts. These artifacts can occur anywhere, such as in parts of the cuff pressure waveform detected during patient pulses, as well as parts detected between patient pulses. However, artifacts that do not occur during patient pulses can render blood pressure determinations more difficult even though pressure readings during the periods between pulses may not be particularly relevant to blood pressure determinations. Therefore, this disclosure appreciates that identifying relevant parts of the cuff pressure waveform can improve blood pressure measurement by facilitating exclusion of artifacts occurring during the less relevant parts of the cuff pressure waveform.

Since the relevant parts of the cuff pressure waveform considered by a blood pressure monitor are tied in time with the heart beating, the cuff pressure waveform can be gated for its relevant segments by electrical signals such as ECG, or blood flow as measured by ultrasound, pulse oximetry measured by light, or other similar techniques. The relevant portion of the cuff pressure waveform can thereby be isolated for processing by the blood pressure monitor, while excluding unrelated artifacts between segments or perhaps offsets within a segment.

Furthermore, adding sensors such as a microphone, stethoscope, or accelerometer on a cuff can assist oscillometric NIBP processing both by enabling detection of pulses for gating purposes, and by enabling identification and removal of motion artifacts from the pressure waveform. Additional sensor(s) can therefore mitigate a limitation of the primary pressure sensor used for oscillometric blood pressure processing.

One example supplemental sensor can include an acoustic sensor such as a microphone or stethoscope. An acoustic sensor can be used in connection with blood pressure readings, pulse detection, and/or detection of noise and corresponding likely vibration or other motion in the environment. For example, an acoustic sensor can be used for blood pressure by placing a microphone or a microphone enabled stethoscope type device over the brachial artery in the antecubital fossa inside a cuff. The acoustic sensor can be used to detect the five phases of Korotkoff sounds which can be used to determine human blood pressure. The phase I Korotkoff sound is a sharp tapping sound that indicates the systolic blood pressure. The phase V Korotkoff sound occurs when all sound disappears and indicates the diastolic blood pressure. The phase I Korotkoff sound can correspond for example to an initial turbulent blood pulse among turbulent blood pulses in the limb of the subject; and the phase V Korotkoff sound can identify a final turbulent blood pulse among the turbulent blood pulses in the limb of the subject.

When an acoustic sensor is deployed along with a blood pressure monitor, oscillometric NIBP processing can be adapted to use an acoustic signal recorded in real-time during blood pressure measurement to identify when at least phase I and V Korotkoff sounds occur. The phase I and V Korotkoff sounds can be correlated to systolic and diastolic points in the measurement timeline and can provide an additional reference point for oscillometric NIBP processing to refine and finalize blood pressure readings, especially when motion is present.

An acoustic sensor can also optionally be used for pulse detection. Acoustic sensors may advantageously provide a more motion tolerant pulse detection than the pulse signal employed by oscillometric measurement. Furthermore, an acoustic sensor can also optionally be used for detection of background noise such as loud voices, banging noises, vehicle acceleration or other noises indicative of likely motion. Points in time that correspond to likely motion can optionally be flagged as artifacts corresponding to likely corrupted portions of the blood pressure signal.

Another example supplemental sensor can include an accelerometer, such as an inertial measurement unit (IMU) or other three-axis accelerometer device. The accelerometer can be placed on a cuff to measure acceleration on the cuff. NIBP processing can be adapted to subtract pressure noise signals generated solely from acceleration (caused by motion) from other measured pressure signals. This can effectively reject motion artifacts from NIBP measurements.

To calculate a pressure noise value generated solely from the measured acceleration, a NIBP signal processor may for example transform three signal vectors from the three accelerometer axes into one combined signal in scalar form. As motion impacts pulse signals and acceleration signals in a similar way, both the measured pressure signal and the acceleration signal have a similar pattern. Example techniques can reduce or minimize the error between these two signals in order to calculate the pressure noise vector caused by motion artifacts.

An accelerometer can also be used to detect excessive motion that exceeds a threshold motion value. NIBP processing can be motion-aware and avoid attempting to determine blood pressure when measurement is impossible due to motion exceeding the maximum threshold.

Implementations of the present disclosure will now be described with reference to the accompanying figures.

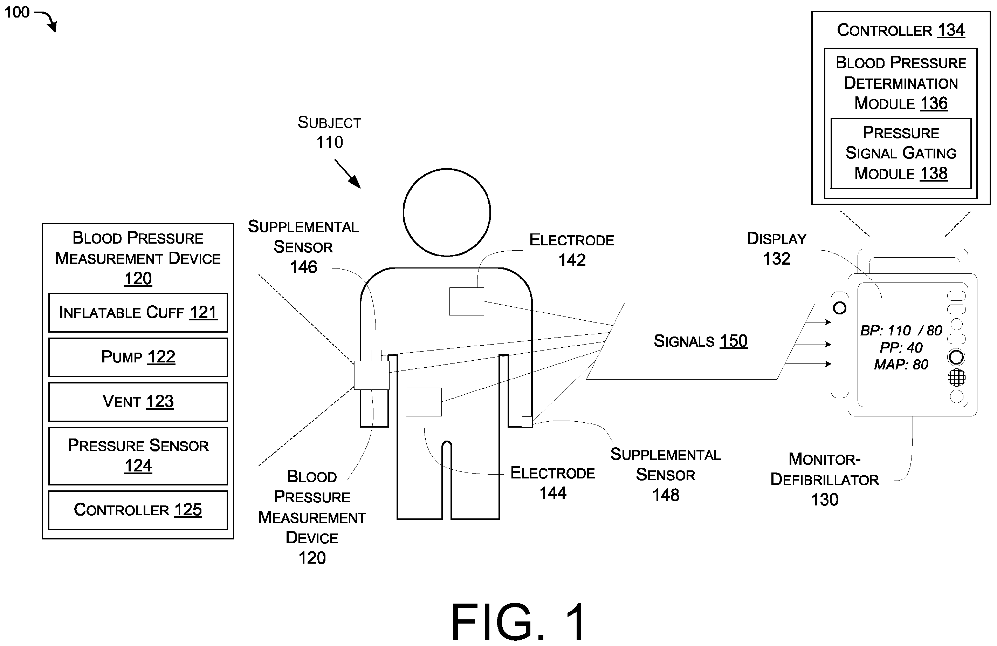

FIG. 1 illustrates an example environment 100 for determining blood pressure of a subject 110 using the blood pressure measurement gating techniques disclosed herein. In some examples, the subject 110 is a patient in a clinical environment such as a hospital, clinic, or ambulance. In some cases, the subject 110 is a patient in a non-clinical environment, such as a rescue scene. For instance, the subject 110 may have experienced a medical emergency (e.g., cardiac event, loss-of-consciousness, acute injury, etc.) in an out-of-hospital environment, such as in a school, a workplace, an airport terminal, or a public space. In some cases, the subject 110 is in a home environment. In various examples, it may be beneficial to assess the blood pressure of the subject 110.

FIG. 1 further illustrates a blood pressure measurement device 120, a monitor-defibrillator 130, electrodes 142, 144, a supplemental sensor 146, and a supplemental sensor 148. The blood pressure measurement device 120 can include, e.g., an inflatable cuff type blood pressure measurement device. The blood pressure measurement device 120 can include, e.g., an inflatable cuff 121, a pump 122, a vent 123, a pressure sensor 124, and a controller 125.

The monitor-defibrillator 130 can include, e.g., a display 132 adapted to present measurements, including blood pressure measurements, pertaining to the subject 110. The monitor-defibrillator 130 can also include computing hardware and software such as a processor and memory, which are not illustrated in FIG. 1 for simplicity of illustration. The monitor-defibrillator 130 can further include a controller 134 including a blood pressure determination module 136. The blood pressure determination module 136 can include a pressure signal gating module 138 adapted to perform the pressure gating techniques described herein.

The monitor-defibrillator 130 can be adapted to connect to, or may include, external sensors and devices, such as the blood pressure measurement device 120, the electrodes 142, 144, the supplemental sensor 146, and the supplemental sensor 148. The monitor-defibrillator 130 can receive signals 150 from external sensors and devices and can process the signals 150 and present resulting data, e.g., the blood pressure data, on the display 132.

In an example according to FIG. 1, the blood pressure measurement device 120 can be installed on the limb of the subject 110, e.g., by wrapping the inflatable cuff 121 around the limb of the subject 110. The blood pressure measurement device 120 can optionally be communicatively coupled with the monitor-defibrillator 130, e.g., via a wired or wireless connection. A blood pressure measurement can be initiated by the subject 110 or by an operator, e.g., an emergency responder, nurse, doctor, or other medical service provider.

In response to a blood pressure measurement initiation, the controller 125 can activate the pump 122 to inflate the inflatable cuff 121, followed by deactivating the pump 122 and activating the vent 123 to deflate the inflatable cuff 121. As the inflatable cuff 121 is inflating, sustained in an inflated state, and subsequently deflating, the controller 125 can sample and record pressure data by detecting, via the pressure sensor 124, the pressure within the inflatable cuff 121. Moreover, the controller 125 can also optionally sample and record secondary data via the supplemental sensor 146. The supplemental sensor 146 can include, e.g., an acoustic sensor, an accelerometer, an ultrasound sensor, a pulse oximeter, a blood flow sensor, or a sensor of any other type as may be desired to carry out the techniques described herein. The supplemental sensor 146 can be attached or integrated into the blood pressure measurement device 120 or can include a separate device with a separate housing and separate communication connection to the monitor-defibrillator 130.

The controller 125 can be configured to send signals 150 to the monitor-defibrillator 130, and the signals 150 can encode the pressure data collected via the pressure sensor 124 as well as, optionally, the secondary data collected via the supplemental sensor 146. Similarly, the monitor-defibrillator 130 can receive signals 150 from the electrodes 142, 144 which encode ECG data.

Furthermore, the monitor-defibrillator 130 can receive signals 150 from additional sensors, such as the supplemental sensor 148. The signals 150 from the supplemental sensor 148 can optionally encode further secondary data. In various implementations, the supplemental sensor 148 is configured to detect a parameter (e.g., physiological parameter) of the subject 110. Examples of parameters include a blood oxygenation (e.g., regional oxygenation, cerebral oxygenation, pulse oxygenation, etc.), an acceleration, a physical orientation, an electrical signal (e.g., an EEG, an ECG, an EMG, etc.), a sound (e.g., an ambient sound, a sound of blood flowing in at least one blood vessel, etc.), a blood flow parameter (e.g., a volumetric blood flow, a blood velocity, a pulse wave velocity, etc.), or any combination thereof. In various cases, the secondary data indicates one or more samples of the detected parameter. The supplemental sensor 148 can include, for instance, an oxygenation sensor (e.g., a pulse oximeter), an accelerometer, a gyroscope, an electrode, a microphone, an interferometric blood flow detector, an ultrasound transducer, or any combination thereof, in some implementations. Alternatively, the supplemental sensor 148 can include any other desired sensor type.

The blood pressure determination module 136 at the controller 134 can be configured to initiate and control the measurement operations performed by the blood pressure measurement device 120, as well as to determine the subject 110 blood pressure using the information received via the signals 150. The blood pressure determination module 136 can furthermore be configured to apply the pressure signal gating module 138 to gate received pressure data as described herein to improve blood pressure determinations.

In general, the pressure signal gating module 138 can use ECG data from the electrodes 142, 144, or other secondary data from the supplemental sensor 146 or the supplemental sensor 148 to determine portions of received pressure data to be removed from further consideration and processing (referred to herein as negative gating) or to determine portions of the received pressure data to be retained for further consideration and processing (referred to herein as positive gating).

Pressure data removed by the pressure signal gating module 138 can include, e.g., data which does not occur during patient pulses, and/or data which occurs during subject 110 movement or otherwise higher (e.g., greater than a threshold) environmental noise conditions, as may be determined based on ECG data or other secondary data. Conversely, pressure data retained by the pressure signal gating module 138 can include, e.g., data which occurs during patient pulses and/or data which occurs during a period without subject 110 movement or otherwise reduced (e.g., lower than a threshold) environmental noise conditions, as determined based on ECG data or other secondary data indicative of movement of the subject 110.

Gated pressure data generated by the pressure signal gating module 138 can optionally be stored and then processed by the blood pressure determination module 136 to determine blood pressure measurements such as systolic blood pressure, diastolic blood pressure, pulse pressure, and MAP. The gated pressure data can include lower noise and fewer artifacts than ungated pressure data, and as a result, blood pressure measurements determined by the blood pressure determination module 136 based on the gated blood pressure data can be more accurate and reliable that determinations based on ungated blood pressure data.

In various alternative arrangements, the controller 134 can be configured to perform some or all of the functions described herein in connection with the controller 125. For example, the controller 134 can be configured to operate the pump 122 and vent 123 to inflate and deflate the inflatable cuff 121, and the controller 134 can be configured to operate the pressure sensor 124 and the supplemental sensor 146 to sample pressure data and/or secondary data. Alternatively, any of the various functions of the controller 134 can optionally be incorporated into the controller 125 in some implementations.

The secondary data collected from the supplemental sensor 146, the electrodes 142, 144, and/or the supplemental sensor 148 can be applied by the controller 134 in one or more ways. First, the secondary data can be used by the blood pressure determination module 136 in connection with blood pressure determinations. For example, the secondary data can include acoustic data that indicates phase I and V Korotkoff sounds, and the timing of the phase I and V Korotkoff sounds can be used to determine systolic blood pressure and diastolic blood pressure. Detecting Korotkoff sounds can involve digital signal processing. A sensitive microphone and a signal amplifier can detect acoustic signals. A software processing component can filter out noise and isolate the frequency band(s) associated with Korotkoff sounds (20-300 Hz). Phase I (characterized by the onset of tapping sounds) and Phase V (marked by the disappearance of sound) can be identified by analyzing acoustic signal's power features, such as short-term energy.

Second, the secondary data can be used by the pressure signal gating module 138 to determine pulse timing information, and the pulse timing information can be used to gate pressure data gathered at the pressure sensor 124. Pulse timing information can be determined by any of several different sensor types using any of several techniques, and this disclosure is not limited to any particular technique.

In one example, the supplemental sensor 146 can include an acoustic sensor which detects pulse sounds, such as the sounds of blood pulses flowing in one or more blood vessels of the subject 110. The pressure signal gating module 138 can use the pulse sounds and timing thereof to determine pulse timing information, which can be used to gate pressure data.

In another example, the electrodes 142, 144 can gather ECG data indicating the contraction muscle at a first location on the subject 110, e.g., at the heart. In various cases, QRS complexes within the ECG data indicate heartbeats of the subject 110, which produce pulses of blood that extend throughout the circulatory system of the subject 110. Due to the delay in time between the contraction of the heart of the subject 110, and the time at which a corresponding pulse reaches an extremity of the subject 110 (e.g., the limb around which the blood pressure measurement device 120 is disposed), the pressure signal gating module 138 can be configured to determine an appropriate delay value to add to an identified heartbeat time, in order to determine pulse timing at the blood pressure measurement device 120.

Similarly, the supplemental sensor 148 may include an oxygenation sensor, such as a pulse oximeter which gathers plethysmograph data including pulse information at a finger of the subject 110. The pressure signal gating module 138 can be configured to determine an appropriate delay value to add or subtract from the time of the gathered pulse information, in order to determine pulse timing at the blood pressure measurement device 120. For instance, a delay value associated with delay at the supplemental sensor 148 can be used, optionally along with other information, to determine the times at which pulses of blood are flowing through the limb on which the inflatable cuff 121 is disposed. The times of pulses at inflatable cuff 121 can in turn enabling the identification of time intervals in the pressure waveform that correspond to systole and diastole. By identifying these time intervals, the pressure data gathered via pressure sensor 124 can be gated in order to measure the diastolic and systolic blood pressure more efficiently, without consideration of artifacts that may occur outside of the time intervals.

Third, the secondary data can be used by the pressure signal gating module 138 to determine artifact timing information, and the artifact timing information can be used to gate pressure data gathered at the pressure sensor 124. For instance, by identifying a time interval when the subject 110 is in motion, the blood pressure measurement device 120 may be prevented from analyzing a portion of the pressure waveform that includes an artifact caused by the motion. This gating process can prevent the blood pressure measurement device 120 from erroneously estimating a blood pressure parameter of the subject 110. Artifact timing information can be determined by any of several different sensor types using any of several techniques, and this disclosure is not limited to any particular technique.

In one example, the supplemental sensor 146 can include an accelerometer which detects movement. The pressure signal gating module 138 can use the detected movements and timing thereof to determine artifact timing information, which can be used to gate pressure data.

In another example, the supplemental sensor 146 can include an acoustic sensor which detects environmental noises such as shouts, bangs, engine noises, or any other environmental noises. Such environmental noises are likely correlated with subject 110 movements and corresponding corruption of pressure data. The pressure signal gating module 138 can use the detected environmental noises and timing thereof to determine artifact timing information, which can be used to gate pressure data.

After blood pressure data is gated by the pressure signal gating module 138, the blood pressure determination module 136 can be configured to use the resulting gated blood pressure data to determine blood pressure measurements. In some implementations, the blood pressure determination module 136 can be configured to generate an oscillometric waveform, and the oscillometric waveform can be relied on for blood pressure measurement determinations such as systolic blood pressure, diastolic blood pressure, pulse pressure, and/or MAP. The determined measurements can be output by the controller 134, e.g., by displaying them on a display 132, or by sending signals representative of the determined measurements other devices. The monitor-defibrillator 130 can be configured to display, via the display 132, blood pressure measurements determined according to the techniques described herein.

The monitor-defibrillator 130 can optionally include numerous other functions in addition to the blood pressure measurement functions described herein. For example, the monitor-defibrillator 130 can receive pulse data from the electrodes 142, 144 and can be configured to control or deliver defibrillation and optionally chest compressions to the subject 110. One example monitor-defibrillator 130 is the LIFEPAK 35^{®} made by STRYKER^{®} Corporation of Portage, Michigan, USA.

One example supplemental sensor 146 can include a flow sensor which detects blood flow. As used herein, the terms "blood flow," "blood flow parameters," and their equivalents, may refer to one or more physiological parameters indicative of a movement of blood through a blood vessel. For example, the term "blood velocity" may refer a change in position with respect to time (i.e., distance per time, such as meters per second) of one or more blood cells moving in a blood vessel. The term "blood speed" may refer to a magnitude of a velocity (i.e., distance per time) of one or more blood cells moving in a blood vessel. The term "velocity profile," for example, may refer to a velocity of blood in a blood vessel with respect to an area defined within the blood vessel, such as a velocity of blood in a blood vessel with respect to a location along a cross-section of the blood vessel. The term "flow rate," and its equivalents, may refer to a volume or mass of blood that passes a boundary (e.g., a cross-section of a blood vessel) with respect to time. The terms "net flow," "net flow volume," and their equivalents, may refer to a volume or mass of blood that passes a boundary during a discrete time interval, such as during a cardiac cycle. A net flow volume can be calculated by integrating a flow rate over the time interval. Unless otherwise specified explicitly or by context, the term "blood flow" may refer to blood velocity, blood speed, velocity profile, flow rate, net flow, or any other flow-related parameter.

Blood flow periodically changes based on a cardiac cycle of the subject 110. During systole, the heart muscle contracts and propels the blood through vasculature of the subject 110. During diastole, the heart muscle relaxes and fills with the blood. The cardiac cycle of the subject 110 is repeated for each heartbeat. The activation of the heart muscle during the cardiac cycle produces an electrical signal that can be detected via an ECG. If the subject 110 does not have an arrhythmia or other medical condition, the cardiac cycle produces cyclical changes in the blood flow and blood pressure with respect to time. Notably, the resultant changes in blood flow and blood pressure may be slightly delayed compared to the actions of the heart of the subject 110, depending on the distance along the vasculature of the subject 110 between the heart and the sensor location.

The blood flow in the subject 110 exhibits a first peak during systole and a second peak during diastole. In various cases, the blood flow has a greater first peak during systole than the second peak during diastole.

According to various implementations, the supplemental sensor 146, which may be coupled with the blood pressure measurement device 120 or the monitor-defibrillator 130, can be configured to sample blood flow multiple times during the cardiac cycle of the subject 110. In some cases, the supplemental sensor 146 samples the blood flow at one or more sampling rates, such as 5 Hz, 10 Hz, 50 Hz, 100 Hz, 200 Hz, 1 kHz, 2 kHz, 3 kHz, 4 kHz, 5 kHz, 10 kHz, or the like. In some cases, the supplemental sensor 146 samples the blood flow during multiple cardiac cycles of the subject 110. According to various cases, the supplemental sensor 146 or the monitor-defibrillator 130 can be configured to generate a waveform of the blood flow over time for each cardiac cycle.

In some examples, the supplemental sensor 146 can perform laser or ultrasound Doppler velocimetry in order to detect the blood velocity. In various cases, the supplemental sensor 146 can include or be coupled with one or more interferometric sensors. For example, the supplemental sensor 146 can output light beams (e.g., coherent light beams) that are split (e.g., by one or more mirrors) prior to transmission through the skin. The supplemental sensor 146 may detect the blood velocity in a blood vessel by comparing a return of the first beam and a return of the second beam to the split beams generated from the first incident beam and the second incident beam. In some implementations, the blood pressure measurement device 120 or the monitor-defibrillator 130 includes, or is coupled to, an ultrasound transducer configured to emit an incident ultrasound beam toward the blood vessel and detect a reflection of the ultrasound beam from blood in the blood vessel. In various cases, a difference in phase and/or frequency between the incident ultrasound beam and the reflection of the ultrasound beam is indicative of the velocity of the blood.

In various examples, the supplemental sensor 146, the blood pressure measurement device 120, and/or the monitor-defibrillator 130 can be configured to detect a pulse pressure of the subject 110. The term "pulse pressure" may refer to a difference between a systolic blood pressure and a diastolic blood pressure. For instance, the pulse pressure of the subject 110 is dependent on the maximum change of pressure in a blood vessel that the heart of the subject 110 produces each time it transitions from the end of diastole to the peak of systole. In various cases, the pulse pressure can be calculated as a function of a change in the blood flow over the course of the cardiac cycle as well as the pulse wave velocity of a pulse wave. According to some examples, the pulse pressure is dependent on the change of a velocity of the blood between the systolic phase and the diastolic phase of the cardiac cycle.

In various implementations, the supplemental sensor 146, the blood pressure measurement device 120, and/or the monitor-defibrillator 130 can be configured to detect a mean arterial pressure (MAP) of the subject 110. MAP may refer to an average blood pressure in a blood vessel during a single cardiac cycle. According to various cases, the blood pressure measurement device 120 can calculate the MAP as a function of the mean of the blood flow through a blood vessel during the cardiac cycle.

In particular cases, the blood pressure measurement device 120 can be configured to detect an instantaneous blood pressure in the subject 110. The instantaneous blood pressure may vary over time during the cardiac cycle of the subject 110 and may be sampled multiple times during the cardiac cycle. For instance, samples of the instantaneous blood pressure may be represented as a time-varying waveform. In some cases, the blood pressure measurement device 120 is configured to detect a systolic blood pressure, which is typically equal to a peak blood pressure during the systolic phase of the cardiac cycle. In various implementations, the blood pressure measurement device 120 detects the systolic blood pressure by identifying a maximum of the blood pressure waveform over the cardiac cycle. In some examples, the blood pressure measurement device 120 is configured to detect a diastolic blood pressure, which is typically equal to a minimum blood pressure during the end of the diastolic phase of the cardiac cycle. In some cases, the blood pressure measurement device 120 the diastolic blood pressure by identifying a minimum of the blood pressure waveform over the cardiac cycle.

In various cases, blood pressure metric(s) can be dependent on the size of the subject 110. In particular implementations, the blood pressure metric(s) can depend on factors such as body fat, musculature, or other soft tissues. Physiologically, the size of the subject 110 is related to the load on the heart of the subject 110 that is provided by the vasculature of the subject 110. Various blood pressure metrics described herein (e.g., pulse pressure, MAP, etc.) are related to the vasculature load of the subject 110.

In various examples, the blood pressure measurement device 120 identifies a metric indicative of the size of the subject 110. In some cases, the size is indicated by a height, a weight, a body mass index (BMI), a height-weight ratio (e.g., a height of the subject 110 divided by a weight or mass of the subject 110), a clothing size (e.g., a shirt size, a pants size, a dress size, etc.), a circumference (e.g., of the neck of the subject 110, of the waist of the subject 110, of the wrist of the subject 110, etc.), a volume of at least a portion of the subject 110, a percent body fat of the subject 110, or some other indication.

In some cases, the size indicates whether the subject 110 is pregnant. In some examples, the blood pressure measurement device 120 or the monitor-defibrillator 130 receives an input signal (e.g., from a user, such as a rescuer) that indicates the size of the subject 110. For instance, the user may measure, estimate, or otherwise identify the size of the subject 110. In some implementations, the blood pressure measurement device 120 or the monitor-defibrillator 130 is communicatively coupled to an electronic medical record (EMR) device which may communicate the size of the subject 110 as indicated in an EMR of the subject 110.

In various implementations of the present disclosure, the blood pressure measurement device 120 or the monitor-defibrillator 130 identifies one or more blood pressure metrics of the subject 110 based, at least in part, on the size of the subject 110. For instance, the function utilized by the blood pressure measurement device 120 to calculate pulse pressure or MAP may further depend on the metric indicative of the size of the subject 110. In some examples, a look-up table utilized by the blood pressure measurement device 120 to identify the pulse pressure or MAP may further depend on the size of the subject 110.

According to some examples, the blood pressure measurement device 120 and/or the monitor-defibrillator 130 can be configured to output an indication of blood pressure metric(s). In some cases, the blood pressure measurement device 120 and/or the monitor-defibrillator 130 can include a display configured to visually present the blood pressure metric(s). For instance, the blood pressure measurement device 120 and/or the monitor-defibrillator 130 may display waveforms representative of the blood pressure over the course of the cardiac cycle, such as an oscillometric waveform. In some examples, the blood pressure measurement device 120 and/or the monitor-defibrillator 130 can include a speaker configured to audibly report the blood pressure metric(s). In some implementations, the blood pressure measurement device 120 and/or the monitor-defibrillator 130 can output an alert upon determining that the blood pressure metric(s) are below a first threshold or above a second threshold. The alert, for instance, may be visual or audible.

In some cases, the blood pressure measurement device 120 transmits, to an external device, signals 150 indicating blood pressure data. In some cases, the signals 150 are transmitted over a wireless interface. For instance, the signals 150 may be BLUETOOTH^{™} signals. The signals 150, for instance, encode data indicative of blood pressure measurement values. In some cases, the communication signals 150 include multiple transmissions that collectively report ongoing measurements identified by the blood pressure measurement device 120.

In some examples, the blood pressure measurement device 120 transmits the signals 150 to a monitor-defibrillator 130. For instance, the monitor-defibrillator 130 includes a display 132 configured to visually present blood pressure metric(s). In some examples, the monitor-defibrillator 130 calculates blood pressure metric(s) based on reported characteristic(s), in accordance with the techniques described herein.

In some implementations, the blood pressure measurement device 120 can be configured to determine one or more metrics described herein by taking an average (e.g., median, mean, etc.) of multiple measurements. In some cases, the blood pressure measurement device 120 selectively outputs, or reports, a running average of multiple (e.g., five, ten, etc.) measurements acquired over time. By aggregating the measurements in the form of an average, the quality of the data or metrics output by the blood pressure measurement device 120 may be enhanced.

In various cases, the blood pressure measurement device 120 or the monitor-defibrillator 130 can be configured to determine a quality metric associated with various metrics or measurements described herein. For example, the blood pressure measurement device 120 or the monitor-defibrillator 130 can be configured to determine a variance, Z-score, or other statistic indicative of the quality of a given metric or measurement. In some cases, the blood pressure measurement device 120 or the monitor-defibrillator 130 can refrain from outputting a metric that is associated with a quality metric that is below a predetermined threshold.

In some examples, the blood pressure measurement device 120 or the monitor-defibrillator 130 can output an average of multiple measurements or calculations that are averaged in view of their respective quality metrics. For instance, the blood pressure measurement device 120 or the monitor-defibrillator 130 may calculate five estimates of pulse pressure, taken over five separate instances of the cardiac cycle. The blood pressure measurement device 120 or the monitor-defibrillator 130 may determine a Z-score associated with each of the five estimates in view of each other. Further, the blood pressure measurement device 120 or the monitor-defibrillator 130 may output an average of the estimates of pulse pressure, weighted in view of the respective Z-scores, such that estimates with a lower Z-score are more represented in the average than estimates with a higher Z-score. Various implementations of the present disclosure reduce the impact of noise on the accuracy of reported metrics.

In some implementations, the blood pressure measurement device 120 or the monitor-defibrillator 130 can further indicate a confidence (e.g., the quality metric) associated with any metric it reports. For example, the blood pressure measurement device 120 or the monitor-defibrillator 130 may output an indication of the variance of blood pressure metric measurements used to generate an average blood pressure metric, along with the average blood pressure metric, in order to report a reliability of the average blood pressure metric as reported. In some cases, the blood pressure measurement device 120 or the monitor-defibrillator 130 can display the average blood pressure metric with a color associated with the variance, such that the average blood pressure metric may be displayed in red if it is based off of measurements with a high (e.g., greater than a threshold) variance or the average blood pressure metric may be displayed in green if it is based off of measurements with a low (e.g., lower than a threshold) variance. In some cases, the confidence associated with the reported metric is numerically reported.

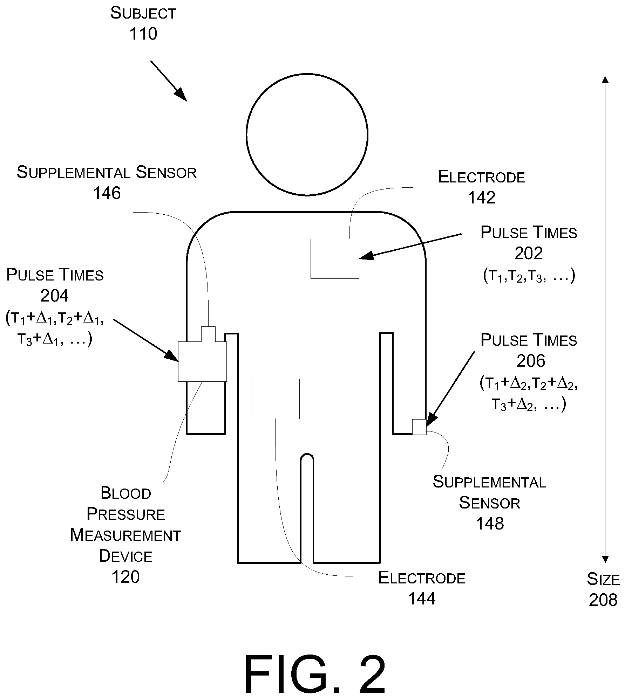

FIG. 2 illustrates example pulse time delays at different sensors deployed on a subject 110. FIG. 2 includes the subject 110 introduced in FIG. 1, as well as the blood pressure measurement device 120, the electrodes 142, 144, the supplemental sensor 146, and the supplemental sensor 148. Furthermore, an example size 208 of the subject 110 is indicated, as well as various example pulse times, including pulse times 202, pulse times 204, and pulse times 206.

FIG. 2 demonstrates that pulses of the subject 110 may be associated with multiple different pulse times at different locations on the subject 110. The pulse times 202 are detected near the heart of the subject 110, the pulse times 204 are detected on a first limb (an arm) of the subject 110 near the blood pressure measurement device 120, and the pulse times 206 are those detected at a second limb (a finger / hand) of the subject 110, near the supplemental sensor 148.

A pulse of blood may originate at the subject's heart, near electrode 142, and the electrode 142 may detect the time of the pulse at a time t₁.For instance, the time t₁ represents the time at which the heart contracts and generates the pulse. The contraction of the heart, for instance, is detected by a shape (e.g., a QRS complex) indicated by an ECG detected by the electrode 142 and electrode 144. A pulse of blood does not travel instantaneously throughout the circulatory system of the subject 110. Accordingly, the same pulse may be slightly delayed by the time it is detected at supplemental sensor 146 or at supplemental sensor 148. The delay at supplemental sensor 146 is represented as Δ₁, and the delay at supplemental sensor 148 is represented as Δ₂. Therefore, the pulse which initiates at time t₁ at the electrode 142 is measured at time t₁ + Δ₁ at the supplemental sensor 146 and is measured at time t₁ + Δ₂ at the supplemental sensor 148. The pulse times 202 include pulse times t₁, t₂, t₃, ..., while the pulse times 204 include pulse times t₁ + Δ₁, t₂ + Δ₁, t₃ + Δ₁, ..., and the pulse times 206 include pulse times t₁ + Δ₂, t₂ + Δ₂, t₃ + Δ₂, .... The delay values Δ₂ and Δ₁ depend on the pulse wave velocity as well as the distance between the subject's heart and a pulse detection location, which is a function of the size 208 of the subject 110.

When pulse timing is used to gate blood pressure measurement data in accordance with this disclosure, the pulse timing information can be adjusted as needed to reflect pulse timing at the blood pressure measurement location. For example, when blood pressure measurement data is collected at the blood pressure measurement device 120 on the arm of the subject, the pulse times 204 can be used to gate the blood pressure measurement data. If pulse timing information is collected at the supplemental sensor 146, no adjustment of the pulse timing information may be needed because the supplemental sensor 146 is near the blood pressure measurement device 120. However, if pulse timing information is collected at the electrode 142, then adjustment of the pulse timing information may be needed, e.g., the pulse timing information can be adjusted by the delay value Δ₁, and the resulting adjusted pulse timing information can be used to gate the blood pressure measurement data.

If pulse timing information is collected at the supplemental sensor 148, then a different adjustment of the pulse timing information may be needed, e.g., the pulse timing information can be adjusted by a delay value (Δ₂ - Δ₃), where Δ₃ is the difference between Δ₁ and Δ₂. Δ₃ can be measured or predicted. The resulting adjusted pulse timing information can be used to gate the blood pressure measurement data.

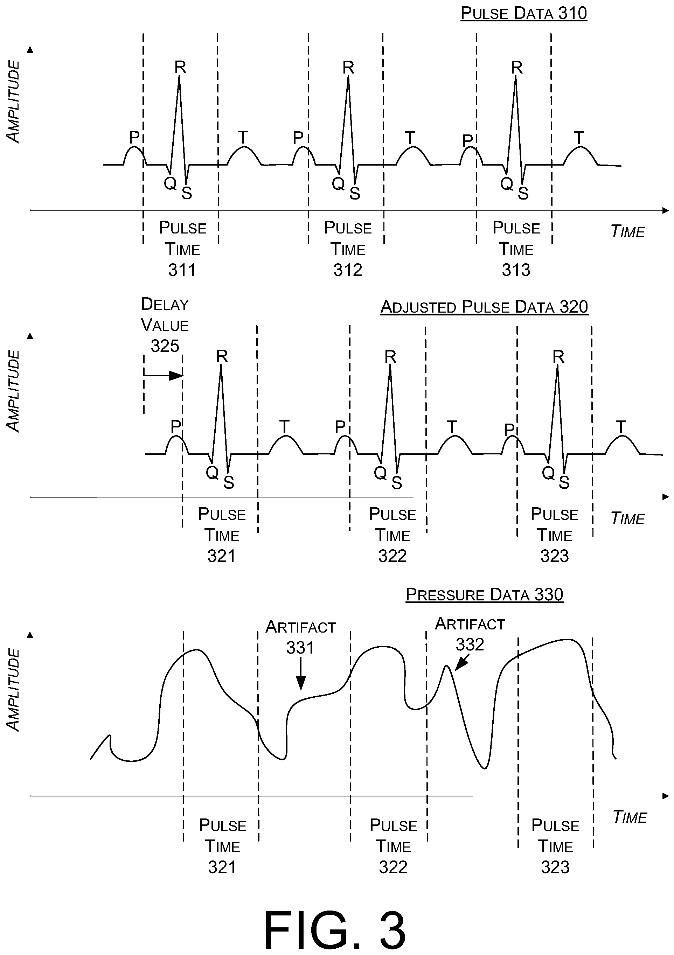

FIG. 3 illustrates example pulse data 310, adjusted pulse data 320 which accounts for a delay, and pressure data 330 which is gated using the adjusted pulse data 320. A pressure signal gating module 138 such as illustrated in FIG. 1 can be configured to use the pulse data 310 and a delay value 325 to generate the adjusted pulse data 320, and to use the adjusted pulse data 320 to process the pressure data 330 so as to gate the pressure data 330.

The pulse data 310 includes three example pulses at different pulse times, e.g., pulse time 311, pulse time 312, and pulse time 313. The pulse data 310 can include, e.g., ECG data gathered from one or more electrodes, such as the electrodes 142 and 144 illustrated in FIG. 1. ECG data can include QRS complexes indicative of heartbeats. Initially, both the atria and ventricles are relaxed (diastole). The P wave represents depolarization of the atria and is followed by atrial contraction (systole). Atrial systole extends until the QRS complex, at which point, the atria relax. The QRS complex represents depolarization of the ventricles and is followed by ventricular contraction. The T wave represents the repolarization of the ventricles and marks the beginning of ventricular relaxation.

Alternatively, the pulse data 310 can include, e.g., acoustic data gathered from a supplemental sensor such as the supplemental sensor 146 or the supplemental sensor 148. In another alternative, the pulse data 310 can include, e.g., plethysmograph data gathered from a supplemental sensor such as the supplemental sensor 146 or the supplemental sensor 148. Of course, acoustic and plethysmograph data will have different features indicative of the cardiac cycle.

A pressure signal gating module 138 such as illustrated in FIG. 1 can be configured to use the pulse data 310 and a delay value 325 to generate the adjusted pulse data 320, as stated above. The delay value 325 can be determined based on the location on the subject 110 of the sensor used to gather the pulse data 310, and the location on the subject 110 of the blood pressure measurement device 120. For example, if the sensor(s) used to gather the pulse data 310 is the electrodes 142, 144 and the blood pressure measurement device 120 is positioned at the location shown in FIG. 1, then the delay value 325 can be Δ₁, as shown in FIG. 2.

In some implementations, the pressure signal gating module 138 can be adapted to determine the delay value 325, while in other implementations, the delay value 325 can be entered by a user of the monitor-defibrillator 130. The delay value 325 can optionally be determined experimentally, by comparing pulse data gathered at a first sensor against pulse data gathered at a second sensor. In another implementation, the delay value 325 can be calculated as a function of subject 110 size, sensor locations, and pulse wave velocity.

The adjusted pulse data 320 can be generated by using the delay value 325 to adjust timing of the pulse data 310. All of the pulse data 310, including the pulse times 311, 312, and 313 can optionally be shifted by the delay value 325. The resulting adjusted pulse data 320 includes delayed pulse times, namely pulse times 321, 322, and 323.

The pulse times 321, 322, and 323 can include any desired portions of the adjusted pulse data 320. In the illustrated example, the pulse times 321, 322, and 323 include time ranges that are centered on the pulse peaks. However, the pulse times 321, 322, and 323 can include, e.g., wider or narrower ranges as may be desired for particular implementations. Alternatively, the ranges can be offset from pulse peaks in some implementations. Pulse times can also optionally include pulse troughs, or some peaks and some troughs.

A pressure signal gating module 138 such as illustrated in FIG. 1 can be configured to use the adjusted pulse data 320, and the pulse times 321, 322, and 323 in particular, to gate the pressure data 330. In an example, the pressure data 330 includes pressure peaks and troughs which overlap the peaks and troughs in the adjusted pulse data 320. However, the pressure data 330 may also include varying amounts of noise. Example occurrences of noise in the pressure data 330 include an artifact 331 and an artifact 332.

The pressure data that occurs during pulse times 321, 322, 323 may be more relevant to blood pressure determinations than pressure data that occurs outside of the pulse times 321, 322, 323. Moreover, the pressure data that occurs outside of the pulse times 321, 322, 323 can include artifacts 331, 332 which can potentially confound or thwart blood pressure determinations based on pressure data 330. Therefore, the pressure signal gating module 138 can be adapted to use the adjusted pulse data 320 to gate the pressure data 330. The pressure signal gating module 138 can for example identify the portions of the pressure data 330 which occur during the pulse times 321, 322, 323 and can extract and optionally store those portions of the pressure data 330, e.g., in a gated pressure data file. The pressure signal gating module 138 can discard or otherwise dis-include the portions of the pressure data 330 which occur outside the pulse times 321, 322, 323. The gated pressure data file can be used for blood pressure determinations by the blood pressure determination module 136.

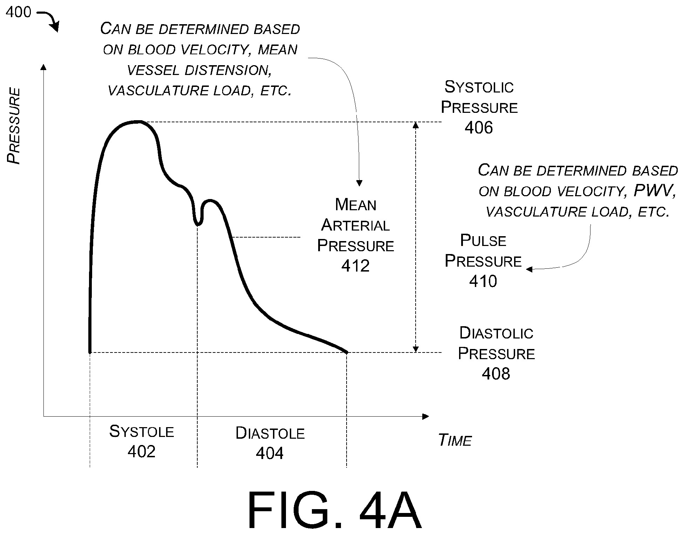

FIG. 4A illustrates an example arterial blood pressure waveform 400. A horizontal axis represents time, and a vertical axis represents pressure. The waveform 400 of FIG. 4A represents blood pressure over the course of a single cardiac cycle, for instance.

The time axis is divided into two regions representing distinct phases of the cardiac cycle. A first phase represents systole 402 and a second phase represents diastole 404. It should be noted that the systole 402 represented in FIG. 4A may be slightly delayed with respect to the heart being in systole, and the diastole 404 represented in FIG. 4A may be slightly delayed with respect to the heart being in diastole, depending on the distance between the heart and the measurement location. The systole 402 phase of FIG. 4A may represent a change in blood pressure that occurs due to the systole phase of the heart, and the diastole 404 phase of FIG. 4A may represent a change in blood pressure that occurs due to the diastole phase of the heart.

During systole 402, the blood pressure in the blood vessel rises to a peak that represents the systolic pressure 406. Subsequently, the pressure decreases until systole 402 ends. During diastole 404, the blood pressure may rise slightly to a second peak that is lower than the systolic pressure 406. Then, the pressure decays to a diastolic pressure 408. A pulse pressure 410 is defined as the difference between the systolic pressure 406 and the diastolic pressure 408. A MAP 412 is defined as the mean of the waveform 400.

It should be noted that various blood pressure metrics described herein can be derived based on a static component of a blood pressure metric (e.g., a component that does not change over the course of the cardiac cycle) as well as a dynamic component of the blood pressure metric (e.g., a component that changes over the course of the cardiac cycle). MAP and pulse pressure are examples of static and dynamic components, but implementations of the present disclosure are not limited to MAP and pulse pressure.

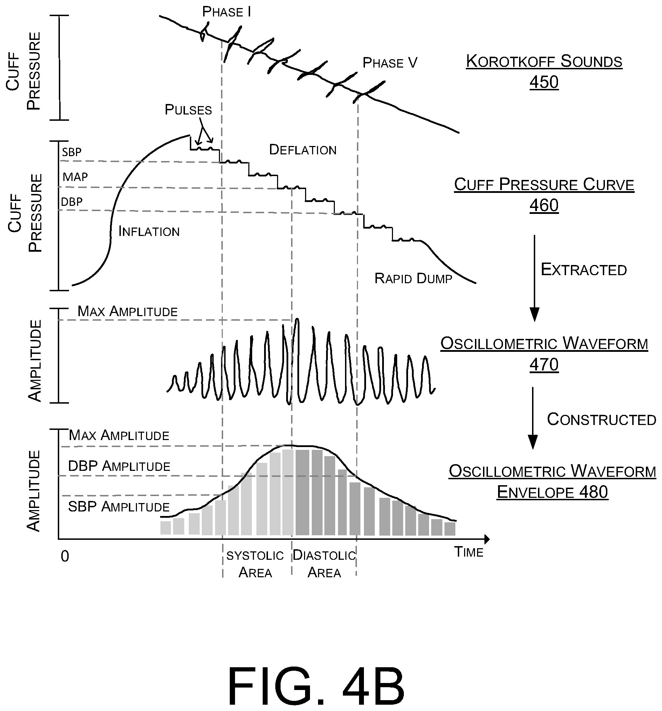

FIG. 4B illustrates an example cuff pressure curve 460, a section of the cuff pressure curve bounded by Korotkoff sounds 450, an oscillometric waveform 470 that can be extracted from the cuff pressure curve 460 and optionally gated according to the techniques described herein, and an oscillometric waveform envelope 480 that can be constructed from the oscillometric waveform 470. The cuff pressure curve 460, oscillometric waveform 470 and/or the oscillometric waveform envelope 480 can be included for example in pressure data 330 illustrated in FIG. 3, which can be gated according to the techniques disclosed herein.

The cuff pressure curve 460 represents the pressure detected within the inflatable cuff 121 over time during a blood pressure measurement cycle. As the cuff deflates, the cuff pressure curve 460 decreases from a maximum inflation pressure, as shown. The Korotkoff sounds 450 can be detected during deflation and correspond to turbulent blood flow in the limb of the subject 110 as the cuff pressure passes through the range between systolic blood pressure (SBP) and diastolic blood pressure (DBP). Phase I of the Korotkoff sounds 450 corresponds to the initial appearance of tapping sounds and indicates the SBP. Phase V of the Korotkoff sounds 450 corresponds to the disappearance of sounds and indicates the DBP. The section of the cuff pressure curve 460 bounded by the Korotkoff sounds 450 represents the clinically relevant portion of the deflation cycle for blood pressure determination.

The oscillometric waveform 470 can be extracted from the cuff pressure curve 460 by filtering or otherwise isolating an oscillatory component of the cuff pressure curve. The oscillometric waveform 470 represents pressure oscillations caused by arterial pulses transmitted to the cuff as blood flows through a compressed artery. The amplitude of the oscillometric waveform 470 varies during deflation, with smaller amplitude oscillations at higher cuff pressures near SBP, increasing oscillation amplitudes as cuff pressure approaches mean arterial pressure (MAP), and decreasing oscillation amplitude as cuff pressure decreases toward DBP. The cuff pressure curve 460 and/or the oscillometric waveform 470 can optionally be gated according to the techniques described herein, to remove artifacts and improve the accuracy of blood pressure determinations.

The oscillometric waveform envelope 480 can be constructed from the oscillometric waveform 470 by identifying the peak amplitudes of the oscillations over time. The oscillometric waveform envelope 480 exhibits a characteristic shape with a maximum amplitude corresponding to MAP. The SBP and DBP can be determined from the oscillometric waveform envelope 480 using ratios or other techniques that identify the cuff pressures at which the oscillation amplitude reaches predetermined fractions of the maximum amplitude. By gating the oscillometric waveform 470 prior to constructing the oscillometric waveform envelope 480, artifacts can be excluded from the envelope construction, thereby improving the reliability of SBP, DBP, and MAP determinations.

In various implementations, the cuff pressure curve 460 and/or the oscillometric waveform 470 can be gated using secondary signals to improve the quality of blood pressure determinations. For example, ECG data from the electrodes 142, 144 can be used to identify pulse intervals, and the pulse intervals can be adjusted by appropriate delay values to account for the travel time of blood pulses from the heart to the limb where the inflatable cuff 121 is disposed. The adjusted pulse intervals can then be used to identify portions of the cuff pressure curve 460 and/or the oscillometric waveform 470 that correspond to arterial pulsations, while excluding portions that may contain motion artifacts or other noise

In another example, acoustic data from a microphone or stethoscope integrated with the blood pressure measurement device 120 can be used to detect Korotkoff sounds 450, and the timing of the Korotkoff sounds 450 can be used to gate the cuff pressure curve 460 and/or the oscillometric waveform 470. By identifying the time intervals during which Korotkoff sounds 450 are detected, the pressure signal gating module 138 can retain portions of the cuff pressure curve 460 and/or the oscillometric waveform 470 that correspond to clinically relevant arterial pulsations while discarding portions that occur outside these intervals.

In a further example, accelerometer data from the supplemental sensor 146 can be used to identify time intervals during which the subject 110 or the inflatable cuff 121 is in motion. The pressure signal gating module 138 can exclude portions of the cuff pressure curve 460 and/or the oscillometric waveform 470 that occur during these motion intervals, thereby removing artifacts caused by movement. The remaining portions of the cuff pressure curve 460 and/or the oscillometric waveform 470 can then be used to construct the oscillometric waveform envelope 480 with improved accuracy.

Additionally, multiple secondary signals can be combined to gate the cuff pressure curve 460 and/or the oscillometric waveform 470. For instance, ECG data can be used to identify pulse intervals for positive gating, while accelerometer data can be used to identify motion intervals for negative gating. The combination of positive and negative gating can result in a gated oscillometric waveform that includes only the portions corresponding to arterial pulsations during periods of minimal motion, thereby maximizing the accuracy of blood pressure determinations derived from the oscillometric waveform envelope 480.

In an example implementation, the pressure signal gating module 138 can be adapted to gate pressure data 330 in a manner that retains sufficient portions of the pressure data 330 for determinations of SBP, DBP, and MAP. For example, the adjusted pulse times 321, 322, 323 can include sufficient ranges to include both systolic and diastolic portions of the pressure data 330. Alternatively, the adjusted pulse times 321, 322, 323 can include times which target different portions cardiac cycle, including some portions which may overlap / target SBP and other times which may overlap / target DPB.

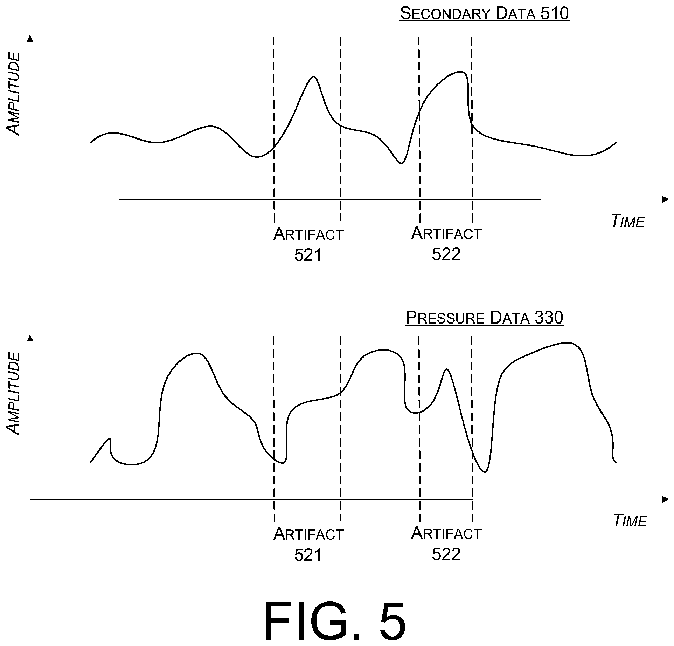

FIG. 5 illustrates example secondary data 510 including features corresponding to likely artifacts 521, 522, and example pressure data 330 which can be adjusted by removing portions identified using the secondary data 510. The pressure signal gating module 138 such as illustrated in FIG. 1 can optionally be configured to use the secondary data 510 to process the pressure data 330 so as to gate the pressure data 330. Gating the pressure data 330 according to the secondary data 510 can be performed either instead of, or in addition to, gating the pressure data 330 according to the adjusted pulse data 320 as described with reference to FIG. 3.

The secondary data 510 can include, e.g., acoustic data gathered from one or more microphone or stethoscope type sensors, which can be included in the supplemental sensor 146 or the supplemental sensor 148. Alternatively, secondary data 510 can include, e.g., accelerometer data gathered from one or more accelerometer type sensors, which can be included in the supplemental sensor 146 or the supplemental sensor 148. Acoustic and accelerometer data are provided as examples and the secondary data 510 can also include other types of data such as video, pulse, temperature, pressure, breath rate, or other types of sensors.

In an example wherein the secondary data 510 includes acoustic data, the secondary data 510 can represent for example the volume of sounds in the environment 100 of the subject 110. The artifact 521 and the artifact 522 may represent, e.g., high amplitude acoustic events such as shouts, bangs, or loud engine noises. These types of events are likely also correlated with movement of the subject 110 which may corrupt blood pressure measurement data.

In an example wherein the secondary data 510 includes accelerometer data, the secondary data 510 can represent for example acceleration as measured at a limb of the subject 110. The artifact 521 and the artifact 522 may represent, e.g., rapid acceleration events such as kicks, convulsions, or other movement. These types of events are likely to corrupt blood pressure measurement data.

In an example, the pressure signal gating module 138 can be configured to identify the artifacts 521, 522 in the secondary data 510, e.g., by identifying higher amplitude portions of the secondary data 510. For example, portions of the secondary data 510 that exceed a threshold amplitude can be identified as artifacts 521, 522. Alternatively, the pressure signal gating module 138 can be configured to identify amplitude peaks in the secondary data 510, and the pressure signal gating module 138 can designate artifacts 521, 522 as ranges centered on the peaks and extending to either side thereof. In another alternative, the pressure signal gating module 138 can be configured to identify any patterns or features of the secondary data 510 which are found to be correlated with likely movement of the subject 110 or corruption of the pressure data 330.

After the artifacts 521, 522 are identified in the secondary data 510, the artifacts 521, 522 can be used to gate the pressure data 330. For example, the pressure signal gating module 138 can be configured to remove portions of the pressure data 330 that occur during the time ranges of the artifacts 521, 522. The remaining pressure data 330 can optionally be stored, e.g., in a measurement file, as gated blood pressure data. The blood pressure determination module 136 can be configured to determine subject 110 blood pressure based on the gated blood pressure data.

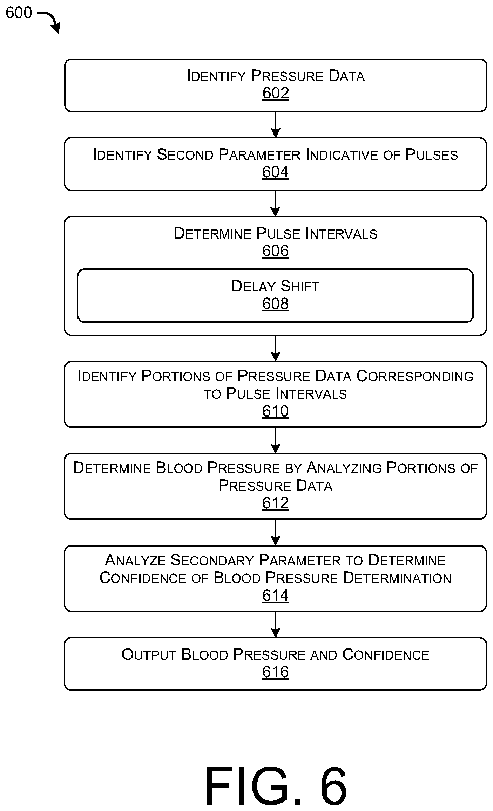

FIG. 6 illustrates an example process 600 for gating blood pressure measurements by a secondary signal to noninvasively determine the blood pressure of a subject. In various examples the process 600 is performed by an entity including the blood pressure measurement device 120, the monitor-defibrillator 130, a medical device, a processor, a further (e.g., an additional) computing device, or any combination thereof.

At operation 602, the entity identifies pressure data, e.g., data indicating a pressure in a cuff configured to be disposed around a limb of a subject. In an implementation, the monitor-defibrillator 130 can be used by an operator to initiate a blood pressure measurement via the blood pressure measurement device 120. The blood pressure measurement device 120 can inflate and deflate the inflatable cuff 121 and can use the pressure sensor 124 to make cuff pressure measurements. The pressure measurements can be sent to the monitor-defibrillator 130. The controller 134 can activate the blood pressure determination module 136 which can identify the pressure measurements for processing to make blood pressure determinations, including processing by the pressure signal gating module 138.

At operation 604, the entity can identify data indicating a secondary parameter indicative of pulses in a limb of the subject 110. For example, the entity can include the monitor-defibrillator 130 and the various devices coupled thereto, including a sensor, such as the electrode 142, the electrode 144, the supplemental sensor 146, or the supplemental sensor 148. The sensor can be configured to detect the secondary parameter indicative of pulses in a limb of the subject 110. The sensor measurements can be sent to the monitor-defibrillator 130. The controller 134 can activate the blood pressure determination module 136 which can identify the sensor measurements for processing to make blood pressure determinations, including processing by the pressure signal gating module 138.

In an example, the secondary parameter can include sounds detected at a limb of the subject 110, e.g., by a microphone or stethoscope type sensor. In another example, the secondary parameter can include an ECG of the subject 110, e.g., as detected at the electrodes 142, 144. A further example secondary parameter can include a movement of the limb of the subject 110 as may be detected by an accelerometer or video camera. In a still further example, the secondary parameter can include a blood oxygenation of a second limb of the subject 110, e.g., as detected at the supplemental sensor 148, wherein blood pressure measurements are made at the blood pressure measurement device 120 disposed on a first limb of the subject 110.

At operation 606, the entity can determine pulse intervals. For example, the pressure signal gating module 138 can analyze the data indicating the secondary parameter, identified via operation 604, in order to determine one or more pulse intervals of the pulses in the limb of the subject 110. Pulse interval determination at operation 606 can optionally include applying delay shift at operation 608, in order to shift pulse interval timing so that it more accurately reflects pulse timing as experienced at the site of the blood pressure measurements.

In an example implementation, when the secondary parameter includes an ECG of the subject 110, the pressure signal gating module 138 can be configured to determine, by analyzing the data indicating the secondary parameter, one or more pulse intervals of the pulses in the limb of the subject 110 by detecting one or more heartbeat intervals by detecting QRS complexes in the ECG, and determining the one or more pulse intervals by shifting the one or more heartbeat intervals by one or more delays, the one or more delays corresponding to travel times of the one or more pulses from the heart of the subject 110 to the limb of the subject 110, e.g., the limb at which the pressure signals are measured. The one or more delays can be based on a pulse wave velocity (PWV) of the subject 110 or an approximation thereof, such as a typical PWV in humans, optionally based on age, size, gender, or other variables.

In another example implementation, the secondary parameter can be blood oxygenation measured at a second limb of the subject 110, e.g., at the secondary sensor 148. A first limb can be an arm of the subject 110 equipped with the blood pressure measurement device 120, while the second limb can be a finger of the subject 110 equipped with a pulse oximeter. The pressure signal gating module 138 can be configured to determine, by analyzing the data indicating the secondary parameter, one or more first pulse intervals of the pulses in the arm of the subject 110 by determining, by analyzing the data indicating the blood oxygenation of the finger of the subject 110, one or more second pulse intervals of the pulses in the finger of the subject 110, and then determining the one or more first pulse intervals (at the arm of the subject 110) based on the one or more second pulse intervals. The one or more second pulse intervals may be delayed with respect to the one or more first pulse intervals, as the finger is further from the subject's heart than the arm.

At operation 610, the entity can identify one or more portions of the pressure data, identified at operation 602, corresponding to the one or more pulse intervals determined at operation 606. For example, the pressure signal gating module 138 can perform a gating operation on data indicating the pressure in the cuff, by including portions of the pressure data having time ranges that match the determined pulse intervals and dis-including portions of the pressure data outside the pulse interval time ranges. The determined pulse intervals used for gating can optionally be delay shifted according to operation 608. The pressure signal gating module 138 can optionally store or otherwise mark or indicate the identified portions of the pressure data, for subsequent use in connection with determining blood pressure measurements.

At operation 612, the entity can determine a blood pressure of the subject 110 by analyzing the gated pressure data, namely, the one or more portions of the data indicating the pressure in the cuff corresponding to the one or more pulse intervals. The blood pressure determination module 136 can be adapted to use the gated portions of the pressure data for blood pressure determinations. The blood pressure determinations can include, e.g., a systolic blood pressure determination, a diastolic blood pressure determination, a pulse pressure determination, a MAP determination, or any other blood pressure measurement.

At operation 614, the entity can optionally analyze the data indicating the secondary parameter to determine a confidence of the blood pressure determination made at operation 612. For example, the blood pressure determination module 136 can be adapted to analyze a noise, irregularity, or variance of the data indicating the secondary parameter. Higher noise, irregularity, or variance can reduce the confidence in the blood pressure determination, while lower noise, irregularity, or variance can increase the confidence in the blood pressure determination. Systolic blood pressure determinations, diastolic blood pressure determinations, pulse pressure determinations, and/or MAP determinations may be associated with a same confidence determination or different confidence determinations. A confidence score can optionally be determined as a percentage, e.g., a percentage between 0-100%, with higher confidence being assigned higher percentage scores. Alternatively, confidence can be scored as a number, e.g., a number between 1-5, with 1 being low confidence and 5 being high confidence.

At operation 616, the entity can cause an output device, e.g., the display 132, to output an indication of the blood pressure, as determined at operation 612. The entity may output a systolic blood pressure determination, a diastolic blood pressure determination, a pulse pressure determination, a MAP determination, or any other indication of blood pressure. The indication may include a number value, a graphical / pictorial output, and/or a color output such as a green, yellow, or red color. The entity may furthermore cause the output device to output an indication of the confidence of the blood pressure of the subject 110, as determined at operation 614. The indication of confidence may also include a number value, a graphical / pictorial output, and/or a color output such as a green, yellow, or red color.

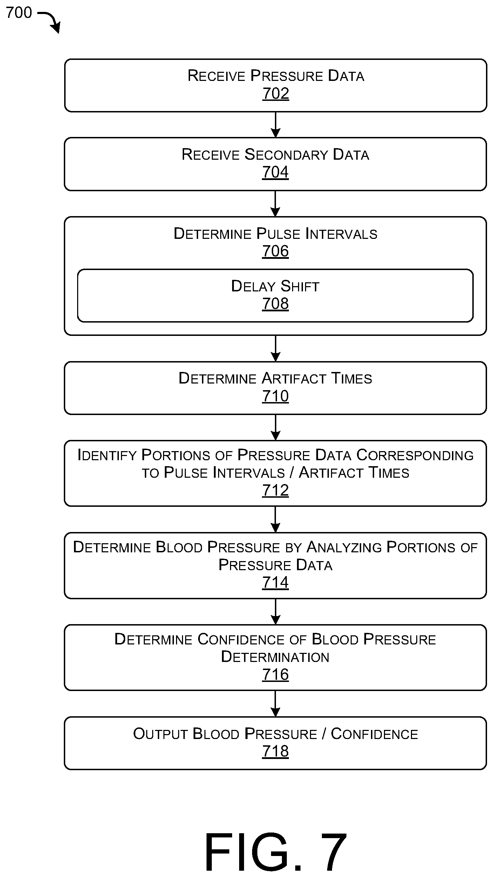

FIG. 7 illustrates another example process 700 for gating blood pressure measurements by a secondary signal to noninvasively determine the blood pressure of a subject. The process 700, in numerous examples, is performed by an entity including the blood pressure measurement device 120, the monitor-defibrillator 130, a medical device, a processor, a further (e.g., an additional) computing device, or any combination thereof. The entity can also include, e.g., a pressure sensor configured to detect the pressure in an inflatable cuff 121, two or more electrodes or other secondary sensors configured to generate secondary data by detecting a secondary parameter, and an output device such as the display 132.

At operation 702, the entity can receive pressure data indicating a pressure in a cuff, e.g., the inflatable cuff 121, configured to be disposed around a limb of a subject 110. For example, the monitor-defibrillator 130 can receive pressure data from the blood pressure measurement device 120. The pressure data can indicate a measurement identifier and can include pressures and corresponding time values. The received pressure data can be stored for subsequent processing.

At operation 704, the entity can receive secondary data, e.g., data from the electrodes 142, 144 or the supplemental sensors 146, 148. The secondary data can optionally indicate pulses in the subject 110 or can indicate other data such as ambient noise or movement of the subject 110, as described herein. For example, the secondary data can optionally include sounds detected from at least one limb of the subject 110. Alternatively, the secondary data can include data indicating a movement of at least one limb of the subject 110.

In another example, the secondary data can include a blood oxygenation of a second limb of the subject which indicates pulses in the second limb of the subject. For example, the secondary data can be received from a pulse oximeter that implements the supplemental sensor 148, which is on a second limb different from the first limb where the blood pressure measurement device 120 is installed.

In another example, the secondary data can include an ECG of the subject 110, and the pressure signal gating module 138 can be configured to detect heartbeats of the subject 110 by detecting QRS complexes in the ECG.

At operation 706, the pressure signal gating module 138 can determine pulse intervals based on the secondary data received at operation 704. The pulse intervals can include, e.g., e.g., pulse peaks, pulse troughs, or other intervals including desired portions of the subject's cardiac cycle. The pulse intervals can span any desired time ranges of the cardiac cycle. Each pulse interval can be bounded by a pulse interval start time and a pulse interval stop time.

At operation 708, a delay shift can optionally be applied to adjust the timing of the pulse intervals. For example, the pressure signal gating module 138 can predict that the pulses in the limb of the subject 110 occur after heartbeats, by delays corresponding to travel times of the pulses from a heart of the subject 110 to the limb of the subject 110. The pressure signal gating module 138 can optionally be configured to determine the delays based on a pulse wave velocity (PWV) of the subject 110.

At operation 710, the pressure signal gating module 138 can optionally determine artifact times in the secondary data that was received at operation 704. Like pulse intervals, artifact times can optionally include time ranges bounded by a start time and a stop time. The artifact times can span over acute / high amplitude noise events which are likely to corrupt pressure data. For example, the pressure signal gating module 138 can determine artifact times associated with the artifacts 521, 522 illustrated in FIG. 3.

In some instances, the entity can perform both of operations 706 and 710. In other instances, the entity can perform one of operations 706 or 710. Both of operations 706 and 710 yield time information that can be used to gate received pressure data, and so either or both may be used to improve blood pressure determinations.

At operation 712, the entity can identify portions of the pressure data corresponding to the pulse intervals (determined at operation 706) or the artifact times (determined at operation 710), or both. In an example, the pressure signal gating module 138 can identify, by analyzing the secondary data, a portion of the pressure data indicating the pressure in the cuff during the pulses in the limb of the subject 110. The portion of the pressure data indicating the pressure in the cuff during the pulses in the limb of the subject 110 may omit a portion of the pressure data indicating the pressure in the cuff during interpulse intervals in the limb of the subject 110.

In another example, the pressure signal gating module 138 can identify, by analyzing the secondary data, a portion of the pressure data indicating the pressure in the cuff that is outside the artifact times determined at operation 710. This portion of the pressure data can be stored or marked for use in connection with further processing to determine blood pressure.

At operation 714, the entity can determine a blood pressure of the subject 110 by analyzing the portion(s) of the pressure data identified at operation 712. The blood pressure determination module 136 can use the gated portions of the pressure data for blood pressure determinations. The blood pressure determinations can include, e.g., a systolic blood pressure determination, a diastolic blood pressure determination, a pulse pressure determination, a MAP determination, or any other blood pressure measurement.

At operation 716, the entity can optionally determine a confidence of the blood pressure of the subject, determined at operation 714, by analyzing the secondary data received at operation 704. As described herein, the blood pressure determination module 136 can be adapted to analyze a noise, irregularity, or variance of the secondary data. Higher noise, irregularity, or variance can reduce the confidence in the blood pressure determination, while lower noise, irregularity, or variance can increase the confidence in the blood pressure determination. Systolic blood pressure determinations, diastolic blood pressure determinations, pulse pressure determinations, and/or MAP determinations may be associated with a same confidence determination or different confidence determinations. A confidence score can optionally be determined as a percentage, e.g., a percentage between 0-100%, with higher confidence being assigned higher percentage scores. Alternatively, confidence can be scored as a number, e.g., a number between 1-5, with 1 being low confidence and 5 being high confidence.

At operation 718, the entity can output an indication of the blood pressure of the subject 110, optionally along with an indication of the confidence of the blood pressure of the subject 110. The entity can cause an output device, e.g., the display 132, to output an indication of the blood pressure, as determined at operation 714. The entity may output a systolic blood pressure determination, a diastolic blood pressure determination, a pulse pressure determination, a MAP determination, or any other indication of blood pressure. The indication may include a number value, a graphical / pictorial output, and/or a color output such as a green, yellow, or red color. The entity may furthermore cause the output device to output an indication of the confidence of the blood pressure of the subject 110, as determined at operation 716. The indication of confidence may also include a number value, a graphical / pictorial output, and/or a color output such as a green, yellow, or red color.

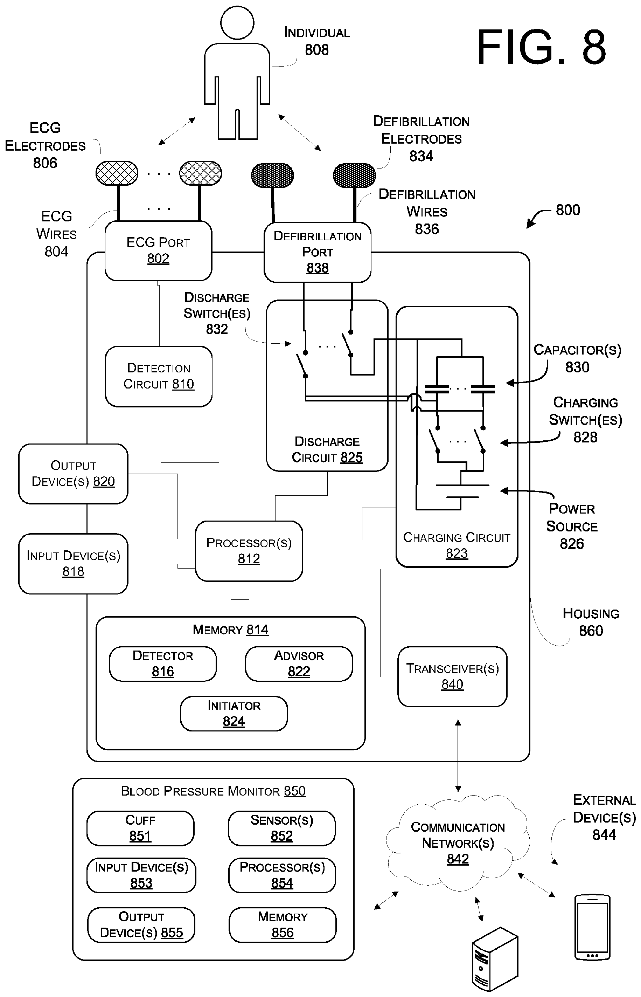

FIG. 8 illustrates an example of an external defibrillator 800 configured to perform various functions described herein. For example, the external defibrillator 800 can implement the monitor-defibrillator 130 described above with reference to FIG. 1.

The external defibrillator 800 includes an ECG port 802 connected to multiple ECG wires 804. In some cases, the ECG wires 804 are removeable from the ECG port 802. For instance, the ECG wires 804 are plugged into the ECG port 802 via connectors. The ECG wires 804 are connected to ECG electrodes 806, respectively. In various implementations, the ECG electrodes 806 are disposed on distinct locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the distinct locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 810 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG wires 804. In some cases, the detection circuit 810 includes one or more analog filters configured to filter noise and/or artifact from the electrical signals. The detection circuit 810 includes an analog-to-digital circuit (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage (or current) across a pair of the ECG electrodes 806 and detects a resultant current (or voltage) between the pair of the ECG electrodes 806. The impedance is generated based on the applied signal (voltage or current) and the resultant signal (current or voltage). In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance."

The detection circuit 810 provides the ECG signal and/or the impedance signal to one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 814 is volatile (such as random access memory (RAM)), nonvolatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and pulseless ventricular tachycardia (VT). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters (e.g., one or more blood pressure parameters), CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. In some examples, the processor(s) 812 executing the initiator 824 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

In an implementation according to this disclosure, the processor(s) 812 and the memory 814 can implement the controller 134 introduced in FIG. 1. For example, the blood pressure determination module 136 and the pressure signal gating module 138 can be stored or loaded into the memory 814 and can cause the processor(s) 812 to perform the various operations disclosed herein.

The processor(s) 812 is operably connected to a charging circuit 823 and a discharge circuit 825. In various implementations, the charging circuit 823 includes a power source 826, one or more charging switches 828, and one or more capacitors 830. The power source 826 includes, for instance, a battery. The processor(s) 812 initiates a defibrillation shock by causing the power source 826 to charge at least one capacitor among the capacitor(s) 830. For example, the processor(s) 812 activates at least one of the charging switch(es) 828 in the charging circuit 823 to complete a first circuit connecting the power source 826 and the capacitor to be charged. Then, the processor(s) 812 causes the discharge circuit 825 to discharge energy stored in the charged capacitor across a pair of defibrillation electrodes 834, which are in contact with the individual 808. For example, the processor(s) 812 deactivates the charging switch(es) 828 completing the first circuit between the capacitor(s) 830 and the power source 826 and activates one or more discharge switches 832 completing a second circuit connecting the charged capacitor 830 and at least a portion of the individual 808 disposed between defibrillation electrodes 834.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation wires 836. The defibrillation wires 836 are connected to a defibrillation port 838, in implementations. According to various examples, the defibrillation wires 836 are removable from the defibrillation port 838. For example, the defibrillation wires 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 840 includes other wireless modems, such as a modem for engaging in WI-FI^{®}, WIGIG^{®}, WIMAX^{®}, BLUETOOTH^{®}, or infrared communication over the communication network(s) 842.

The defibrillator 800 is configured to transmit and/or receive data (e.g., ECG data, blood pressure data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis.

According to various cases, the external device(s) 844 can include a blood pressure monitor 850 configured to non-invasively detect a blood pressure of the individual 808. In some examples, the blood pressure monitor 850 can implement the blood pressure measurement device 120 described above with reference to FIG. 1. The blood pressure monitor 850 includes, for example, a cuff 851, sensor(s) 852, input device(s) 853, processor(s) 854, output device(s) 855, and memory 856. The illustrated components of the blood pressure monitor 850 can implement the components of the blood pressure measurement device 120. For example, the cuff 851 can implement the inflatable cuff 121, the pump 122, and the vent 123. The sensor(s) 852 can implement the pressure sensor 124.

The blood pressure monitor 850, in various cases, includes at least one processor 854 configured to execute instructions stored in memory 856, thereby performing various functions described herein. In some examples, the processor(s) 854 is configured to inflate and deflate the cuff 851, gather pressure data via sensor(s) 852, and send the pressure data to the defibrillator 800. Any type of processor and/or memory described elsewhere herein can be suitable as the processor(s) 854 and/or memory 856 of the blood pressure monitor 850.

In various cases, the blood pressure monitor 850 further includes one or more transceivers to transmit and/or receive communication signals with the transceiver(s) 840 of the defibrillator 800 over the communication network(s) 842. In some examples, the blood pressure monitor 850 further includes at least one input device 853 (e.g., a button, a touchscreen, a microphone, etc.) and at least one output device 855 (e.g., a display, a speaker, etc.). Any type of input device and/or output device described herein can be included in the input device(s) 853 and/or output device(s) 855. In some examples, the input device(s) 853 includes a supplemental sensor such as the supplemental sensor 146 illustrated in FIG. 1, and the blood pressure monitor 850 can be configured to send data gathered via the supplemental sensor 146 to the defibrillator 800.

In particular examples, the blood pressure monitor 850 transmits an indication of a blood pressure of the individual 808 to the defibrillator 800 via a communication signal. In some cases, the defibrillator 800 performs various functions based on the blood pressure reported by the blood pressure monitor 850. For example, the detector 816 may include instructions to detect pulseless VT in response to detecting VT in the ECG of the individual 808 as well as in response to determining that the blood pressure detected by the blood pressure monitor 850 is below a threshold blood pressure. In various cases, the advisor 822 includes instructions that cause the processor(s) 812 to output a recommendation to administer an electrical shock to the individual 808 in response to detecting the pulseless VT. In some cases, the output device(s) 820 of the defibrillator 800 output an indication of the blood pressure detected by the blood pressure monitor 850.

According to various cases, the external device(s) 844 can include additional sensors, such as the supplemental sensors 146, 148. The defibrillator 800 can be configured to gather secondary data via the supplemental sensors 146, 148 during blood pressure measurement cycles performed by the blood pressure monitor 850, and the defibrillator 800 can use the secondary data to gate pressure data retrieved via blood pressure monitor 850, as described herein.

In various implementations, the external defibrillator 800 also includes a housing 860 that at least partially encloses other elements of the external defibrillator 800. For example, the housing 860 encloses the detection circuit 810, the processor(s) 812, the memory 814, the charging circuit 823, the transceiver(s) 840, or any combination thereof. In some cases, the input device(s) 818 and output device(s) 820 extend from an interior space at least partially surrounded by the housing 860 through a wall of the housing 860. In various examples, the housing 860 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, discharges the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

### EXAMPLE CLAUSES

The following Clauses provide various examples of the present disclosure:
1. A medical device, including: a cuff configured to be disposed around a limb of a subject; a pump configured to inflate the cuff during an inflation time; a vent configured to deflate the cuff during a deflation time; a pressure sensor configured to detect a pressure in the cuff; a detection circuit configured to detect an electrocardiogram (ECG) of the subject; a display; and a processor configured to: detect heartbeat times of the subject by detecting QRS complexes in the ECG of the subject; determine pulse times in the limb of the subject by adding delays to the heartbeat times; identify pulse time gated data indicating the pressure in the cuff during the pulse times; determine a blood pressure of the subject by analyzing the pulse time gated data; and cause the display to visually present an indication of the blood pressure of the subject.
2. The medical device of clause 1, the heartbeat times being first heartbeat times, the QRS complexes being first QRS complexes, the pulse times being first pulse times, and the medical device further including a microphone integrated with the cuff and configured to detect sounds indicative of pulses in the limb of the subject at second pulse times, wherein the processor is further configured to: detect second heartbeat times by detecting second QRS complexes in the ECG of the subject; and determine the delays by comparing the second pulse times and the second heartbeat times.
3. The medical device of any of clauses 1 to 2, the limb being a first limb, the medical device further including an oximetry sensor configured to be disposed on a second limb of the subject and to detect a plethysmograph of blood in the second limb of the subject, and wherein the processor is further configured to detect the pulse times further by identifying peaks in the plethysmograph.
4. A medical device, including: an output device; and a processor configured to: identify data indicating a pressure in a cuff configured to be disposed around a limb of a subject; identify data indicating a secondary parameter indicative of pulses in the limb of the subject; determine, by analyzing the data indicating the secondary parameter, one or more pulse intervals of the pulses in the limb of the subject; identify a portion of the data indicating the pressure in the cuff corresponding to the one or more pulse intervals; determine a blood pressure of the subject by analyzing the portion of the data indicating the pressure in the cuff corresponding to the one or more pulse intervals; and cause the output device to output an indication of the blood pressure.
5. The medical device of clause 4, wherein the secondary parameter includes an ECG of the subject, and wherein the processor is configured to determine, by analyzing the data indicating the secondary parameter, the one or more pulse intervals of the pulses in the limb of the subject by: detecting one or more heartbeat intervals by detecting QRS complexes in the ECG; and determining the one or more pulse intervals by shifting the one or more heartbeat intervals by one or more delays, the one or more delays corresponding to travel times of the one or more pulses from the heart of the subject to the limb of the subject.
6. The medical device of clause 5, wherein the processor is configured to determine the one or more delays based on a pulse wave velocity (PWV) of the subject.
7. The medical device of any of clauses 4 to 6, wherein the secondary parameter includes sounds detected at the limb of the subject.
8. The medical device of any of clauses 4 to 7, wherein the secondary parameter includes a movement of the limb of the subject.
9. The medical device of any of clauses 4 to 8, the limb being a first limb, wherein the secondary parameter includes a blood oxygenation of a second limb of the subject.
10. The medical device of clause 9, the one or more pulse intervals being one or more first pulse intervals, wherein the first limb is an arm of the subject and the second limb is a finger of the subject, and wherein the processor is configured to determine, by analyzing the data indicating the secondary parameter, the one or more first pulse intervals of the pulses in the arm of the subject by: determining, by analyzing the data indicating the blood oxygenation of the finger of the subject, one or more second pulse intervals of the pulses in the finger of the subject; and determining the one or more first pulse intervals based on the one or more second pulse intervals, the one or more second pulse intervals being delayed with respect to the one or more first pulse intervals.
11. The medical device of any of clauses 4 to 10, wherein the processor is further configured to: determine a confidence of the blood pressure of the subject by analyzing the data indicating the secondary parameter; and cause the output device to output an indication of the confidence of the blood pressure of the subject.
12. The medical device of any of clauses 4 to 11, further including a sensor configured to detect the secondary parameter.
13. A method, including: identifying data indicating a pressure in a cuff configured to be disposed around a limb of a subject; identifying data indicating a secondary parameter indicative of pulses in the limb of the subject; determining, by analyzing the data indicating the secondary parameter, one or more pulse intervals of the pulses in the limb of the subject; identifying a portion of the data indicating the pressure in the cuff corresponding to the one or more pulse intervals; determining a blood pressure of the subject by analyzing the portion of the data indicating the pressure in the cuff corresponding to the one or more pulse intervals; and outputting an indication of the blood pressure.
14. The method of clause 13, wherein the secondary parameter includes an ECG of the subject, and wherein determining, by analyzing the data indicating the secondary parameter, the one or more pulse intervals of the pulses in the limb of the subject includes: detecting one or more heartbeat intervals by detecting QRS complexes in the ECG; and determining the one or more pulse intervals by shifting the one or more heartbeat intervals by one or more delays, the one or more delays corresponding to travel times of the one or more pulses from the heart of the subject to the limb of the subject.
15. The method of clause 14, wherein determining the one or more delays includes determining a pulse wave velocity (PWV) of the subject.
16. The method of any of clauses 13 to 15, wherein the secondary parameter includes sounds detected at the limb of the subject.
17. The method of any of clauses 13 to 16, wherein the secondary parameter includes a movement of the limb of the subject.
18. The method of any of clauses 13 to 17, the limb being a first limb, wherein the secondary parameter includes a blood oxygenation of a second limb of the subject.
19. The method of clause 18, the one or more pulse intervals being one or more first pulse intervals, wherein the first limb is an arm of the subject and the second limb is a finger of the subject, and wherein determining, by analyzing the data indicating the secondary parameter, the one or more first pulse intervals of the pulses in the arm of the subject includes: determining, by analyzing the data indicating the blood oxygenation of the finger of the subject, one or more second pulse intervals of the pulses in the finger of the subject; and determining the one or more first pulse intervals based on the one or more second pulse intervals, the one or more second pulse intervals being delayed with respect to the one or more first pulse intervals.
20. The method of any of clauses 13 to 19, further including: determining a confidence of the blood pressure of the subject by analyzing the data indicating the secondary parameter; and causing the output device to output an indication of the confidence of the blood pressure of the subject.
21. A medical device, including: a cuff configured to be disposed around a limb of a subject; a pump configured to inflate the cuff during an inflation time; a vent configured to deflate the cuff during a deflation time; and a pressure sensor configured to detect a pressure in the cuff during the inflation time and the deflation time; a detection circuit configured to detect an electrocardiogram (ECG) of the subject during the inflation time and the deflation time; a display; and a processor configured to: detect heartbeat times of the subject by detecting QRS complexes in the ECG; predict blood pulse times in the limb of the subject by adding delays to the heartbeat times; receive pressure data indicating the pressure in the cuff during the inflation time and the deflation time; determine gated data by identifying at least a portion of pressure data indicating the pressure in the cuff during the blood pulse times; determine a systolic blood pressure and a diastolic blood pressure of the subject by analyzing the gated data; and cause the display to visually present an indication of the systolic blood pressure and the diastolic blood pressure of the subject.
22. The medical device of clause 21, further including: a microphone integrated with the cuff and configured to detect a sound at the cuff; wherein the processor is further configured to: detect, by analyzing the sound at the cuff during the deflation time, turbulent blood pulses in the limb of the subject, and wherein the processor is further configured to determine the gated data based on the turbulent blood pulses in the limb of the subject.
23. The medical device of clause 22, wherein the processor is further configured to: identify an initial turbulent blood pulse among the turbulent blood pulses in the limb of the subject; and identify a final turbulent blood pulse among the turbulent blood pulses in the limb of the subject, and wherein the processor is configured to determine the systolic blood pressure further based on a pressure in the cuff during the initial turbulent blood pulse, and wherein the processor is configured to determine the diastolic blood pressure further based on a pressure in the cuff during the final turbulent blood pulse.
24. A medical device, including: an output device; and a processor configured to: receive pressure data indicating a pressure in a cuff configured to be disposed around a limb of a subject; receive secondary data indicating pulses in the limb of the subject; identify, by analyzing the secondary data, a portion of the pressure data indicating the pressure in the cuff during the pulses in the limb of the subject; determine a blood pressure of the subject by analyzing the portion of the pressure data; and output an indication of the blood pressure of the subject.
25. The medical device of clause 24, wherein the secondary data includes an electrocardiogram (ECG) of the subject, and wherein the processor is further configured to: detect heartbeats of the subject by detecting QRS complexes in the ECG; and predict that the pulses in the limb of the subject occur after the heartbeats by delays corresponding to travel times of the pulses from a heart of the subject to the limb of the subject.
26. The medical device of clause 25, wherein the processor is configured to determine the delays based on a pulse wave velocity (PWV) of the subject.
27. The medical device of any of clauses 24 to 26, wherein the portion of the pressure data indicating the pressure in the cuff during the pulses in the limb of the subject omits a portion of the pressure data indicating the pressure in the cuff during interpulse intervals in the limb of the subject.
28. The medical device of any of clauses 24 to 27, wherein the secondary data includes sounds detected from at least one limb of the subject.
29. The medical device of any of clauses 24 to 28, wherein the secondary data includes data indicating a movement of at least one limb of the subject.
30. The medical device of any of clauses 24 to 29, the limb being a first limb, wherein the secondary data indicating pulses in the limb of the subject includes a blood oxygenation of a second limb of the subject.
31. The medical device of any of clauses 24 to 30, wherein the processor is further configured to: determine a confidence of the blood pressure of the subject by analyzing the secondary data; and cause the output device to output an indication of the confidence of the blood pressure of the subject.
32. The medical device of any of clauses 24 to 31, wherein the medical device further includes: a pressure sensor configured to detect the pressure in the cuff; and a secondary sensor configured to generate the secondary data by detecting a secondary parameter.
33. A method, including: receiving pressure data indicating a pressure in a cuff configured to be disposed around a limb of a subject; receiving secondary data indicating pulses in the limb of the subject; identifying, by analyzing the secondary data, a portion of the pressure data indicating the pressure in the cuff during the pulses in the limb of the subject; determining a blood pressure of the subject by analyzing the portion of the pressure data; and outputting an indication of the blood pressure of the subject.
34. The method of clause 33, wherein the secondary data includes an electrocardiogram (ECG) of the subject, and further including: detecting heartbeats of the subject by detecting QRS complexes in the ECG; and predicting that the pulses in the limb of the subject occur after the heartbeats by delays corresponding to travel times of the pulses from a heart of the subject to the limb of the subject.
35. The method of clause 34, wherein determining the delays is based on a pulse wave velocity (PWV) of the subject.
36. The method of any of clauses 33 to 35, wherein the portion of the pressure data indicating the pressure in the cuff during the pulses in the limb of the subject omits a portion of the pressure data indicating the pressure in the cuff during interpulse intervals in the limb of the subject.
37. The method of any of clauses 33 to 36, wherein the secondary data includes sounds detected from at least one limb of the subject.
38. The method of any of clauses 33 to 37, wherein the secondary data includes data indicating a movement of at least one limb of the subject.
39. The method of any of clauses 33 to 38, the limb being a first limb, wherein the secondary data indicating pulses in the limb of the subject includes a blood oxygenation of a second limb of the subject.
40. The method of any of clauses 33 to 39, further including: determining a confidence of the blood pressure of the subject by analyzing the secondary data; and causing an output device to output an indication of the confidence of the blood pressure of the subject.
41. A medical device, including: a cuff configured to be disposed around a limb of a subject; a pump configured to inflate the cuff during an inflation time; a vent configured to deflate the cuff during a deflation time; and a pressure sensor configured to detect a pressure in the cuff during the inflation time and the deflation time; a detection circuit configured to detect an electrocardiogram (ECG) of the subject during the inflation time and the deflation time; a display; and a processor configured to: detect heartbeat times of the subject by detecting QRS complexes in the ECG; predict blood pulse times in the limb of the subject by adding delays to the heartbeat times; receive pressure data indicating the pressure in the cuff during the inflation time and the deflation time; determine gated data by identifying at least a portion of pressure data indicating the pressure in the cuff during the blood pulse times; determine a systolic blood pressure and a diastolic blood pressure of the subject by analyzing the gated data; and cause the display to visually present an indication of the systolic blood pressure and the diastolic blood pressure of the subject.
42. The medical device of clause 41, further including: a microphone integrated with the cuff and configured to detect a sound at the cuff; wherein the processor is further configured to: detect, by analyzing the sound at the cuff during the deflation time, turbulent blood pulses in the limb of the subject, and wherein the processor is further configured to determine the gated data based on the turbulent blood pulses in the limb of the subject.
43. The medical device of clause 42, wherein the processor is further configured to: identify an initial turbulent blood pulse among the turbulent blood pulses in the limb of the subject; and identify a final turbulent blood pulse among the turbulent blood pulses in the limb of the subject, and wherein the processor is configured to determine the systolic blood pressure further based on a pressure in the cuff during the initial turbulent blood pulse, and wherein the processor is configured to determine the diastolic blood pressure further based on a pressure in the cuff during the final turbulent blood pulse.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can include, consist essentially of, or consist of its particular stated element, step, or component. Thus, the terms "include" or "including" should be interpreted to recite: "include, consist of, or consist essentially of." The transition term "include" or "includes" means has, but is not limited to, and allows for the inclusion of unspecified elements, steps, ingredients, or components, even in major amounts. The transitional phrase "consisting of" excludes any element, step, ingredient, or component not specified. The transition phrase "consisting essentially of" limits the scope of the implementation to the specified elements, steps, ingredients, or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device, including:
an output device; and
a processor configured to:
receive pressure data indicating a pressure in a cuff configured to be disposed around a limb of a subject;
receive secondary data indicating pulses in the limb of the subject;
identify, by analyzing the secondary data, a portion of the pressure data indicating the pressure in the cuff during the pulses in the limb of the subject;
determine a blood pressure of the subject by analyzing the portion of the pressure data; and
output an indication of the blood pressure of the subject.

2. The medical device of claim 1, wherein the secondary data includes an electrocardiogram (ECG) of the subject, and wherein the processor is further configured to:
detect heartbeats of the subject by detecting QRS complexes in the ECG; and
predict that the pulses in the limb of the subject occur after the heartbeats by delays corresponding to travel times of the pulses from a heart of the subject to the limb of the subject.

3. The medical device of claim 2, wherein the processor is configured to determine the delays based on a pulse wave velocity (PWV) of the subject.

4. The medical device of claim 1, wherein the portion of the pressure data indicating the pressure in the cuff during the pulses in the limb of the subject omits a portion of the pressure data indicating the pressure in the cuff during interpulse intervals in the limb of the subject.

5. The medical device of claim 1, wherein the secondary data includes sounds detected from at least one limb of the subject.

6. The medical device of claim 1, wherein the secondary data includes data indicating a movement of at least one limb of the subject.

7. The medical device of claim 1, the limb being a first limb, wherein the secondary data indicating pulses in the limb of the subject includes a blood oxygenation of a second limb of the subject.

8. The medical device of claim 1, wherein the processor is further configured to:
determine a confidence of the blood pressure of the subject by analyzing the secondary data; and
cause the output device to output an indication of the confidence of the blood pressure of the subject.

9. The medical device of claim 1, wherein the medical device further includes:
a pressure sensor configured to detect the pressure in the cuff; and
a secondary sensor configured to generate the secondary data by detecting a secondary parameter.

10. A method, including:
receiving pressure data indicating a pressure in a cuff configured to be disposed around a limb of a subject;
receiving secondary data indicating pulses in the limb of the subject;
identifying, by analyzing the secondary data, a portion of the pressure data indicating the pressure in the cuff during the pulses in the limb of the subject;
determining a blood pressure of the subject by analyzing the portion of the pressure data; and
outputting an indication of the blood pressure of the subject.

11. The method of claim 10, wherein the secondary data includes an electrocardiogram (ECG) of the subject, and further including:
detecting heartbeats of the subject by detecting QRS complexes in the ECG; and
predicting that the pulses in the limb of the subject occur after the heartbeats by delays corresponding to travel times of the pulses from a heart of the subject to the limb of the subject,
optionally wherein determining the delays is based on a pulse wave velocity (PWV) of the subject.

12. The method of claim 10, wherein the portion of the pressure data indicating the pressure in the cuff during the pulses in the limb of the subject omits a portion of the pressure data indicating the pressure in the cuff during interpulse intervals in the limb of the subject.

13. The method of claim 10, wherein the secondary data includes sounds detected from at least one limb of the subject and/or data indicating a movement of at least one limb of the subject.

14. The method of claim 10, the limb being a first limb, wherein the secondary data indicating pulses in the limb of the subject includes a blood oxygenation of a second limb of the subject.

15. The method of claim 10, further including:
determining a confidence of the blood pressure of the subject by analyzing the secondary data; and
causing an output device to output an indication of the confidence of the blood pressure of the subject.
